# EUROPEAN PATENT SPECIFICATION

(11) **EP 1 663 395 B1**
(45) Date of publication and mention of the grant of the patent: **22.07.2009**
(21) Application number: 04782221.8
(22) Date of filing: 26.08.2004
(51) Int. Cl.: A61P 3/10, A61K 31/155, A61K 45/06, A61K 31/366, A61K 31/401, A61K 31/616, A61K 31/4415, A61K 31/714, A61K 31/519, A61K 31/64, A61K 9/00

(54) **MULTI-SYSTEM THERAPY FOR DIABETES, THE METABOLIC SYNDROME AND OBESITY COMPRISING A HYPOGLYCEMIC AGENT**
MULTISYSTEM-THERAPIE FÜR DIABETES, DAS METABOLISCHE SYNDROM UND ADIPOSITAS MIT EINEM HYPOGLYKÄMISCHEN MITTEL
THERAPIE MULTISYSTEMIQUE CONTRE LE DIABETE, LE SYNDROME METABOLIQUE ET L'OBESITE COMPRENANT UN AGENT HYPOGLYCEMIQUE

(30) Priority: 08.09.2003 US 501226 P; 15.06.2004 US 868227
(43) Date of publication of application: 07.06.2006
(73) Proprietor: Folli, Franco, 20135 Milano (IT); Manfredi, Paolo, New York, NY 10012 (US); Gonzales, Gilbert, New York, NY 10021 (US)
(72) Inventor: Folli, Franco, 20135 Milano (IT); Manfredi, Paolo, New York, NY 10012 (US); Gonzales, Gilbert, New York, NY 10021 (US)
(74) Representative: Findlay, Alice Rosemary
(86) International application number: PCT/US2004/027689
(87) International publication number: WO 2005/025673

(56) References cited:
- EP-A- 1 269 860
- WO-A-00/45818
- WO-A-98/10786
- WO-A-20/04091612
- US-A1- 2003 049 314
- US-A1- 2003 092 697
- US-B2- 6 562 807

## Description

### RELATED APPLICATION

This application is related to Provisional U.S. Patent Application Serial No. 60/501,226, filed on September 9, 2003.

### BACKGROUND OF THE INVENTION

Obesity, the Metabolic Syndrome (also referred to as Syndrome X), and Type II Diabetes Mellitus, are very much interrelated. Type II Diabetes Mellitus is caused by a combination of defective insulin secretion and insulin resistance. This results in elevated blood sugar, elevated blood pressure, increased blood lipids and obesity. This determines micro and macro vascular disease with cardiovascular accidents, renal insufficiencies, neuropathy, blindness and higher incidences of infections. Over 120 million people worldwide suffer from diabetes, and 90% of these are Type II Diabetes. Eighty percent of those with diabetes are obese. This has an enormous economic impact in addition to the obvious personal impact on those suffering from this disease.

The Metabolic Syndrome is characterized by individuals having abdominal obesity, high triglycerides, low HDL cholesterol, high blood pressure and high fasting glucose levels. A diagnosis of Metabolic Syndrome is made when three or more of these factors are established. It is believed that as many as 25% of the general population in the United States suffer from Metabolic Syndrome. In addition to being a direct cause of death, those suffering from Metabolic Syndrome have a significantly increased risk of developing Type II Diabetes with all of its complications, as well as other serious cardiovascular complications.

Finally, in the United States, over 61 % of the population is obese or overweight. Fifty-eight million people are overweight, forty million are characterized as obese, and three million are considered morbidly obese.

A somewhat related malady is polycystic ovary syndrome. Polycystic ovary syndrome is characterized by anovulation (irregular or absent menstrual periods) and hyperandrogenism (elevated serum testosterone and androstenedione). Patients with this syndrome may complain of abnormal bleeding, infertility, obesity, excess hair growth, hair loss and acne. Insulin resistance, high blood pressure, high serum lipids, increased risk for cardiovascular disease and increased risk of developing Type II Diabetes are also important features.

Polycystic ovary syndrome is estimated to affect about half as many or approximately 10% of women.

One of the major biochemical features of polycystic ovary syndrome is insulin resistance accompanied by compensatory hyperinsulinemia (elevated fasting blood insulin levels). Hyperinsulinemia has also been associated with high blood pressure and increased clot formation and appears to be a major risk factor for the development of heart disease, stroke and Type II Diabetes.

The medical literature suggests that the endocrinopathy in most patients with polycystic ovary syndrome can be resolved with insulin lowering therapy.

Those people suffering from diabetes generally are treated with a number of different agents, in particular hypoglycemic agents, blood pressure medication, as well as cholesterol-lowering drugs. It is common to use multiple different drugs to treat Type II Diabetes. Several drug combinations have been produced.

Such drug combinations are disclosed in Ikeda U.S. patent 5,952,356, Adjei U.S. patent 6,524,621, Chaudhari U.S. pending application 2003/0219482 A1, Jaen et al U.S. application 2002/0037928 A1; Fine et al U.S. patent 6,376,594, Pierson U.S. patent 6,693,094, Whitcomb U.S. patent 6,01 1,049, Saho et al U.S. patent 6,645,997; Chungi U.S. patent 6,669,955; and Luskey U.S. patent 6,646,004. Further, Liang et al U.S. patent 6,576,256 discloses a combination of a cholesterol-lowering agent, a renin-angiotensin system inhibitor and aspirin. Sethi et al U.S. patent 6,489,345 discloses a combination of E vitamins and hypoglycemic drug, and Doebbner et al U.S. patent 5,847,008 discloses a variety of combinations with acetyl phenols. Other pending applications include 2003/0149111; 2003/01 14469; 2003/0149111; and 2003/0158090.

Many of these combinations do not include a hypoglycemic agent. Further, others will include hypoglycemic agents which are unsuitable for treatment of Metabolic Syndrome or obesity. The biguanides are used to treat diabetes, and can also be useful in treating obesity and Syndrome X. However, other hypoglycemic agent such as the sulfonylureas, the alpha glucosidase inhibitors, the glitazones, and the meglitinides will actually induce hypoglycemia in patients who are not suffering from Type II Diabetes. Biguanides have been combined with these other hypoglycemic agents for treatment of Type II Diabetes. Other combination therapies fail to adequately address all of the comorbidities associated with Type II Diabetes with one medicine. Compliance is a critical problem for proper treatment of diabetes. Only a fraction of patients with diabetes is compliant with prescribed regimens. Individuals required to take five or six different pills, either at once or at different times of the day, are less likely to do so. Thus, reducing the number of pills, or, in other words, combining drugs into a single dosage will greatly improve compliance.

The present invention in one aspect provides a composition for treatment for Type II Diabetes, Syndrome X and obesity comprising a hypoglycemic agent consisting of a biguanide, a HGM CoA reductase inhibitor, and an ACE inhibitor, said composition in a single unit dosage.

The present invention in another aspect provides a composition for treatment of Type II Diabetes consisting of a biguanide hypoglycemic agent, a lipid lowering agent, and an ACE inhibitor, and at least one of the following: an anti-platelet drug, a folate, vitamin B6, vitamin B12, a carotinoids, vitamin A, vitamin C, vitamin D, vitamin E, vitamin K, zinc, polyunsaturated fatty acid, arginin, and an arginin isomer in a single unit dose.

The present invention in a further aspect provides a composition wherein said blood lipid lowering agent is selected from the group consisting of HMG CoA reductase inhibitors, bile acid sequestrants, probucol and fibric acid agents.

The present invention in a further aspect provides a single unit dosage for treating an individual with Type II Diabetes consisting of metformin, an ACE inhibitor, and a HGM CoA reductase inhibitor and at least one or more of aspirin, a folate, one or more vitamins and minerals.

### SUMMARY OF THE INVENTION

The present invention is premised on the realization that a combination therapy, that is, a metabolic pill, effective for treatment of diabetes, the Metabolic Syndrome and obesity as well as their complications, is provided by utilizing a combination of a biguanide hypoglycemic agent with a lipid lowering agent, a blood pressure lowering agent, optionally an anti-platelet agent, and optionally a combination of vitamins and supplements which have been shown to prevent atherosclerosis and infections. In order to provide a single pharmaceutical that can treat all three of these maladies, the hypoglycemic agent cannot be used in combination with other types of hypoglycemic agents, such as the alpha-glucosidase inhibitors, and the like, because of the risk of inducing hypoglycemia. However, when the pharmaceutical is designed to treat only Type II Diabetes, additional hypoglycemic agents can be employed.

A preferred form of the present invention includes metformin as the hypoglycemic agent, simvastatin as the cholesterol lowering agent, and a renin-angiotensin system inhibitor such as lisinopril as the blood pressure lowering agent. The preferred anti-platelet agent is aspirin. These may be combined with one or more of the following: a folate, vitamin B6, B12 and other supplements such as asparginin, beta-carotene, vitamins A, C, D, E, K, and polyunsaturated fatty acids.

The metabolic pill of the present invention can also improve the symptoms of polycystic ovary syndrome, reduce blood pressure, reduce blood lipids and reduce the incidence of developing Type II Diabetes and cardiovascular complications.

The objects and advantages of the present invention will be further appreciated in light of the following detailed description.

### DETAILED DESCRIPTION OF THE INVENTION

The present invention is a therapeutic composition, a metabolic pill or medicine intended to be administered in a single dose application, i.e., all of the pharmaceuticals combined in a single pill, capsule or liquid formulation, which includes a biguanide hypoglycemic agent in combination with additional pharmaceuticals including a cholesterol lowering agent, a blood pressure lowering agent and, optionally, an anti-platelet agent, vitamins and supplements. This composition can be used whenever clinically appropriate to treat Type II Diabetes and, when appropriate, the Metabolic Syndrome and obesity.

The primary component of the pharmaceutical of the present invention is a hypoglycemic biguanide. Metformin is the preferred oral hypoglycemic agent.

When used to treat Type II Diabetes, the biguanide hypoglycemic drug can be combined with other hypoglycemic drugs or replaced altogether by a separate hypoglycemic drug. Other suitable hypoglycemic drugs include sulfonylureas such as glyburide, alpha glucosidase inhibitors such as acarbose, glitazones such as pioglitazone and rosiglitazone, and meglitinides such as repaglinide. Preferred hypoglycemic agents for treatment of Type II Diabetes would include metformin by itself, pioglitazone by itself, metformin in combination with glyburide, metformin in combination with acarbose, metformin in combination with pioglitazone, metformin in combination with pioglitazone and glyburide, and repaglinide plus pioglitazone.

The minimum effective adult dosage of metformin is about 250 mg, administered daily. The dosage is increased to a maximum dosage of 3.5 gm daily. The present combination drug is designed to maintain all of its components within individual drug therapeutic windows as the dosage is escalated from 1 capsule/pill (5 ml liquid) once a day to 5 capsules/pills (25 ml liquid) 3 times a day. A minimum dose is established for each of the individual components that is within the effective dose range for that component for at least a subgroup of patients. If a higher dosage is required, additional medication (i.e., an additional pill) is taken. The dosage of the individual component is established to allow increasing the dosage without exceeding the maximum dosage of any component.

The blood pressure lowering agents of the present invention are renin angiotensin system inhibitors

VIZ. ACE inhibitors which inhibit the conversion of angiotensin I to angiotensin II. ACE inhibitors include sulfhydryl containing ACE inhibitors including captopril and agents that are structurally related to captopril such as fentialpril, pivalopril, zofenopril, and alacepril. Other ACE inhibitors include the dicarboxyl-containing ACE inhibitors including amalopril, lisinopril, benazapril, quinapril, moexipril, ramipril, spirapril, perindopril, indolapril, pentopril, and cilazapril. Phosphorus-containing ACE inhibitors can also be used, such as fosinopril and ACE inhibitors structurally related thereto.

The preferred ACE inhibitors are captopril, silizopril, delapril, analopril, fentiapril, fosinopril, endolapril, lisinopril, perindopril, pivalopril, quinapril, ramipril, spirapirl, trandolapril and zofinopril. Particularly preferred are captopril, enalipril, fosinopril, lisinopril, quinapril, ramipril, and trandolapril. Most preferred are lisinopril and ramipril.

Examples of ACE/NEP inhibitors for use herein include, without limitation, those disclosed in U.S. patents 5,508,272, 5,362,727, 5,366,973, 5,225,401, 4,722,810, 5,223,516, 5,552,397, 4,749,688, 5,504,080, 5,612,359 and 5,525,723.

Preferred are those ACE/NEP inhibitors that are designated as preferred in the above U.S. patents. Particularly preferred are the ACE/NEP inhibitors omapatrilat and MDL100240, disclosed in U.S. patent 5,430,145.

The preferred dosage for each of these components obviously will depend on the particular pharmaceutical. The effective dosage ranges for these compounds are well known

The third component of the present invention is a blood lipid lowering agent. There are many different blood lipid lowering agents. These include, HMG CoA reductase inhibitors, bile acid sequestrants, probucol, and fibric acid agents. The HMG CoA reductase inhibitors include atorvastatin, cerivistatin, fluindostatin, fluvastatin, lovastatin, mevastatin, pravastatin, simvastatin, and velostatin. The preferred HMG CoA reductase inhibitors are simvastatin, pravastatin, lovastatin, and atorvastatin. The bile acid sequestrants include cholestyramine, colestipol, and colesevelam. The fibric acid agents include clofibrate, fenofibrate, and gemfibrozil.

Again, the dosage rate for each of these components will be determined by the particular lipid lowering agent selected. Generally, the minimum dose will be designed for individuals with normal to slightly raised lipid levels.

A further component of the present invention is preferably an anti-platelet drug. The preferred anti-platelet drug is aspirin. Other salicylates can be used such as magnesium salicylate. Further, there are other anti-platelet aggregating agents, such as anagrelide, dipyridamole, clopidogrel, and ticlopidine. Further, cyclooxygenase inhibitors can be used, including nonstearoidal anti-inflammatory drugs such as ibuprofen, sulindac, sulindac sulfide, sulindac sulfone, flurbiprofen, indomethacin, naproxen, meclafenamic acid, and piroxicam. These should be provided in a dosage effective to inhibit platelet degradation.

The invention further may include various vitamins and supplements shown to prevent atherosclerosis and to prevent infections. One preferred vitamin is folic acid, a folate, or folinic acid, commonly referred to as a folate. Suitable folates include 5-methyl tetrahydrofolic acid, tetrahydrofolic acid, and 5-formyl tetrahydrofolic acid.

Vitamin B components should be included such as vitamin B6 (pyridoxine), and vitamin B12. Other vitamins and minerals include beta-carotine and other carotinoids, vitamin A, vitamin C, vitamin D, vitamin E, vitamin K, zinc, polyunsaturated fatty acids, arginin, as well as its isomers.

The individual components of the present invention are preferably combined to form an oral dosage form. This can include tablets, capsules, caplets, solutions, suspensions and/or syrups, as well as granules, beads, powders or pellets that may or may not be encapsulated. Suppository preparations and sublingual preparations, as well as transdermal patches, may also be used.

A preferred general formulation for the present invention would include:

| | |
|---|---|
| metformin | 50-1500 mg |
| simvastatin | 1-80 mg |
| lisinopril | 1-40 mg |

Another preferred general formula includes:

| | |
|---|---|
| metformin | 50-1500 mg |
| simvastatin | 1-80 mg |
| lisinopril | 1-40 mg |
| aspirin | 5-375 mg |
| folic acid | 0.5-1 mg |
| vitamin B6 | 5-40 mg |
| vitamin B12 | 0.05-1 mg |

A preferred specific formula in capsule form includes:

### (Specific Formula 1)

| | |
|---|---|
| metformin | 250 mg |
| aspirin | 12.5 mg |
| simvastatin | 3.5 mg |
| lisinopril | 1.66 mg |
| folic acid | 0.166 mg |
| vitamin B6 | 8.33 mg |
| vitamin B12 | 0.166 mg |

| | |
|---|---|
| methocel E4M as an excipient (QS in gelatin capsule size 1) | |

A liquid formulation would include:

### (Specific Formula 2)

| | |
|---|---|
| metformin | 250 mg |
| aspirin | 12.5 mg |
| simvastatin | 3.5 mg |
| lisinopril | 1.66 mg |
| folic acid | 0.166 mg |
| vitamin B6 | 8.33 mg |
| vitamin B12 | 0.166 mg |

in a fixed oil base comprising 200 mg of silica gel, 5 mg butylated hydroxyteluline, 6.25 mg stevia powder, 0.1 ml of flavor and olive oil forming 5 ml of medicine.

For the treatment of Diabetes Type II, the composition of the present invention will preferably include the biguanide hypoglycemic agent, a lipid lowering agent, ACE inhibitor, and, optionally vitamin B12 compounds, folic acid, vitamin C, arginin, as well as other supplements. Additional hypoglycemic agents can also be employed such as the sulfonylureas, alpha-glucosidase inhibitors, glitazones and meglitinides.

For treatment of Syndrome X, polycystic ovary syndrome, and/or obesity, the composition will include only the biguanide hypoglycemic agent, preferably metformin, and no other type of hypoglycemic agent. The remainder of the formulation should remain the same as the above formulation for Type II Diabetes.

Additional examples of formulations encompassing the present invention are set forth below.

### Formula A

Metformin 250 mg, aspirin 12.5 mg, simvastatin 3.5 mg, lisinopril 1.66 mg, folic acid 0.166 mg, vitamin B6 8.33 mg and vitamin B12 0.166 mg for each capsule or, in a liquid, for each 5cc.

### Formula B

Metformin 50-1000 mg, simvastatin 1-40 mg, lisinopril 1-10 mg, aspirin 5-75 mg, vitamin B6 10-50 mg, vitamin B12 0.2-1 mg, plus folic acid 0.2-1 mg.

### Formula C

Metformin 1000-3000 mg, simvastatin 20-40 mg, ramipril 0.6-1.25 mg, aspirin 35-75 mg, plus polyunsaturated fatty acids 500-1000 mg.

### Formula D

Metformin extended release up to 3500 mg, repaglinide 2 mg (or glimepiride 2 mg), simvastatin 40 mg, lisinopril 10 mg; aspirin 175 mg, vitamin B6 50 mg, vitamin B12 1 mg, plus folic acid 1 mg.

### Formula E

Metformin 500-3500 mg, repaglinide 0.5 mg, simvastatin 40 mg, ramipril 2.5 mg, plus aspirin 81 mg.

### Formula F

Metformin 500-1000 bid or tid, glimepiride 2 mg, simvastatin 20-80 mg, lisinopril 10-30 mg, aspirin 75 mg, vitamin B6 50 mg, vitamin B12 1 mg, plus folic acid 2 mg.

### Formula G

Metformin ER 1000-3000 mg, simvastatin 40 mg, lisinopril 5 mg, atenolol 25 mg, hydrochlorothiazide 25 mg, aspirin 75 mg, vitamin B6 50 mg, vitamin B12 1 mg, plus folic acid 1 mg.

### Formula H

Metformin 1000-3000 mg, repaglinide 0.5 mg, simvastatin 40 mg, lisinopril 5 mg, atenolol 25 mg, hydrochlorothiazide 25 mg, aspirin 75 mg, vitamin B6 50 mg, vitamin B12 1 mg, plus folic acid 1 mg.

### Formula I

Metformin ER 1000-3000, simvastatin 40 mg, lisinopril 5 mg, atenolol 25 mg, hydrochlorothiazide 25 mg; aspirin 75 mg, vitamin B6 50 mg, vitamin B12 1 mg, plus folic acid 1 mg.

### Formula L

Metformin 1000-3000 mg, glimepiride 2 mg, simvastatin 40 mg, lisinopril 5 mg, atenolol 25 mg, hydrochlorothiazide 25 mg, aspirin 75 mg, vitamin B6 50 mg, vitamin B12 1 mg, plus folic acid 1 mg.

### Formula M

Metformin ER 3000 mg, simvastatin 40 mg, ramipril 10 mg, aspirin 75 mg, vitamin B6 25 mg, vitamin B12 1 mg, folic acid 0.8 mg, plus polyunsaturated fatty acids 1000 mg.

### Formula N

Pioglitazone 20 mg metformin 1000 mg, repaglinide 1.25 mg, simvastatin 40 mg, ramipril 10 mg; aspirin 75 mg, vitamin B6 50 mg, vitamin B12 1 mg, folic acid 1 mg, plus polyunsaturated fatty acids 1000 mg.

### EXPERIMENTAL STUDY

To determine preliminary toxicology, normal mice, with a starting weight range of 18.4 -24.4 grams, were randomly assigned to one of four treatment groups, ten mice for each group, and treated once a day for 7 days by gastric gavage with one of 0.2 ml normal saline, 0.015 ml of a placebo comprising calcium carbonate and a fixed oil base in combination with 0.185 ml normal saline, or, 0.015 ml of specific formula 1, plus 0.185 ml normal saline, or, 0.03 ml of specific formula 1 plus 0.17 ml of normal saline. At the end of the treatment the animals were weighed and sacrificed. Blood was obtained for biochemical and hormonal tests. Heart, liver and kidney were frozen at -70° C for further proteomic and enzymatic studies, and the liver was fixed for routine histological examination. On day 7, all animals appeared to be in good health. Biochemical tests did not show signs of liver or kidney toxicity. The weight was lower in both treatment groups 3 and 4 compared to the placebo groups 1 and 2. Leptin levels were decreased in the treatment groups compared to placebos. Glucose and triglycerides were lower and insulin levels were higher in the treatment group. Set forth below are the starting and ending weights of the respective mice, broken down by groups.

### GROUP 1: NS

| **Starting weight (grams)** | **Ending weight (grams)** |
|---|---|
| 22.6 | 22 |
| 22.2 | 21.3 |
| 20.8 | 22 |
| 19 | 10 |
| 21.2 | 20 |
| 21 | 18.9 |
| 22 | 18.7 |
| 20.6 | 21 |
| 21.8 | 22.3 |
| 22.3 | 23 |

### GROUP 2: VEHICLE

| **Starting weight (grams)** | **Ending weight (grams)** |
|---|---|
| 23.5 | 22.9 |
| 21.8 | 20.9 |
| 20.3 | 18.4 |
| 21.7 | 18.6 |
| 20.4 | 18.9 |
| 21.8 | 20 |
| 22.2 | 22.6 |
| 21.3 | 22.3 |
| 20.8 | 21 |
| 23.9 | 23 |

### GROUP 3: MTDMO 0.1 ml

| **Starting weight (grams)** | **Ending weight (grams)** |
|---|---|
| 20.7 | 16.7 |
| 20.9 | 16.2 |
| 20.4 | 15.2 |
| 23.5 | 15 |
| 20.6 | 18 |
| 23 | 16.1 |
| 21.1 | 16.1 |
| 20.8 | 15 |
| 24.4 | 16.6 |
| 21.1 | 16.9 |

### GROUP 4: MTDMO 0.2 ml

| **Starting weight (grams)** | **Ending weight (grams)** |
|---|---|
| 20.6 | 16.3 |
| 19.6 | 15.1 |
| 21 | 12 |
| 21.9 | 14.9 |
| 18.7 | 15.1 |
| 21.8 | 15.9 |
| 19.7 | 13.4 |
| 19.2 | 14.2 |
| 23.6 | 16.3 |
| 21.2 | 14.1 |

As can be seen, Groups 3 and 4 lost substantially more weight than the placebo groups.

The compositions of the present invention are designed to significantly improve compliance, and lower costs. Compliance is often a primary issue, especially as drug regimens become more complex. Type II Diabetes and the Metabolic Syndrome X and obesity, when not responsive to diet and exercise, require complex drug regimen for optimal treatment and prevention of the complications. Improvement of compliance is the primary goal of all effective treatments, especially important with complex regimens. A combination pill such as this will reduce costs and significantly increase the likelihood of compliance.

Particularly for patients with Type II Diabetes, multiple drugs are required. Based on the recommendations of the American Diabetes Association, the cholesterol lowering agent is indicated even if cholesterol level is normal, and an inhibitor of a renin angiotensin system is indicated even if the blood pressure is normal. Aspirin is indicated in patients with Type II Diabetes, even in the absence of previous cardiovascular events. One prescription, such as the above, would cover both diabetes control and prevention of complications improving patients compliance. The availability of one formulation which contains all of the ingredients needed by patients with diabetes will help physicians correctly prescribe for their patients. Furthermore, the cost of a single pill will significantly lower costs compared to the cost of the different pharmaceuticals taken separately.

All the above combinations can be used in combination with insulin. Insulin is frequently required in patients with long standing diabetes mellitus, and oral hypoglycemic agents may lower the insulin requirements, Insulin at high doses may have a proatherogenic effect. The combination drug will, therefore, have multiple benefits compared to insulin alone.

With respect to Metabolic Syndrome, which is not responsive to diet and exercise, the above formulation can slow the evolution toward diabetes, particularly utilizing metformin and the ACE inhibitor. Further, the composition will decrease body weight, using the metformin, and lower the risk of cardiovascular complications employing basically all of the components.

Further, with respect to treatment of obesity, the above composition has been effective to provide weight loss in experimental work. Obese patients are at high risk for diabetes and cardiovascular complications. Metformin and the ACE inhibitors can prevent the development of diabetes. All the above components will help prevent cardiovascular complications. The biguanide hypoglycemic agents, particularly metformin, is the hypoglycemic agent of choice. Metformin does not cause hypoglycemia and is, therefore, the drug of choice for prevention of diabetes in patients and with Metabolic Syndrome and obesity not responding to diet and exercise.

The formulation of the present invention, aside from treating diabetes, Syndrome X and obesity, can be used to treat other maladies such as polycystic ovary syndrome.

This has been a description of the present invention along with the preferred method of practicing the present invention.

## Claims

1. A composition, formulated as a single unit dosage, for use in the treatment of Type II Diabetes, Syndrome X and obesity comprising a hypoglycemic agent consisting of a biguanide, a HGM CoA reductase inhibitor, and an ACE inhibitor, said composition in a single unit dosage.

2. The composition claimed in claim 1 wherein said ACE inhibitor is selected from the group consisting of sulfhydril containing ACE inhibitors, dicarboxyl containing ACE inhibitors and phosphorus containing ACE inhibitors.

3. The composition claimed in claim 1 wherein said statin is selected from the group consisting of simvastatin, pravastatin, lovastatin, and atorvastatin.

4. The composition claimed in claim 1 further comprising at least one of the following: a folate, vitamin B6, vitamin B12, a carotinoids, vitamin A, vitamin C, vitamin D, vitamin E, vitamin K, zinc, polyunsaturated fatty acids, arginin and isomers thereof.

5. The composition claimed in claim 4 wherein said composition includes vitamin B6 and vitamin B12.

6. A composition for use in the treatment of Type II Diabetes consisting of a biguanide hypoglycemic agent, a lipid lowering agent, and an ACE inhibitor, and at least one of the following: an anti-platelet drug, a folate, vitamin B6, vitamin B12, a carotinoids, vitamin A, vitamin C, vitamin D, vitamin E, vitamin K, zinc, polyunsaturated fatty acid, arginin, and an arginin isomer all of which are present in a single unit dose.

7. The composition claimed in claim 6 wherein said hypoglycemic agent is selected from the group consisting of metformin, pioglitazone, metformin plus glyburide, metformin plus acarbose, metformin plus pioglitazone, metformin plus pioglitazone plus gliburide, and repaglinide plus pioglitazone.

8. A composition in a single unit dosage for use in treating an individual diagnosed with at least one of Type II Diabetes, Syndrome X, polycystic ovary syndrome, and obesity comprising a hypoglycemic agent consisting of a biguanide, a lipid lowering agent, and an ACE inhibitor.

9. The composition claimed in either claim 2 or claim 8 wherein said ACE inhibitor is selected from the group consisting of lisinopril and ramipril.

10. The composition claimed in claim 8 wherein said blood lipid lowering agent is selected from the group consisting of HMG CoA reductase inhibitors, bile acid sequestrants, probucol and fibric acid agents.

11. The composition claimed in claim 10 wherein said HMG CoA reductase inhibitor is selected from the group consisting of simvastatin, pravastatin, lovastatin and atorvastatin.

12. The composition claimed in either claim 1 or claim 8 wherein said biguanide hypoglycemic agent is metformin.

13. The composition claimed in claim 12 further comprising an anti-platelet drug.

14. The composition claimed in claim 13 wherein said anti-platelet drug is selected from the group consisting of salicylates, anti-platelet aggregating agents, and cyclooxygenase inhibitors.

15. The composition claimed in claim 14 wherein said salicylate is selected from the group consisting of acetyl salicylic acid and magnesium salicylate.

16. The composition claimed in claim 14 wherein said anti-platelet aggregating agent is selected from the group consisting of anagrelide, dipyridamole, clopidogrel, and ticlopidine.

17. The composition claimed in claim 14 wherein said cyclooxygenase inhibitor is selected from the group consisting of ibuprofen, sulindac, sulindac sulfide, sulindac sulfone, flurbiprofen, indomethacin, naproxen, meclafenamic acid, and piroxicam.

18. The composition claimed in claim 8 wherein said medicine further comprises at least one of the following: a folate, vitamin B6, vitamin B12, a carotinoids, vitamin A, vitamin C, vitamin D, vitamin E, vitamin K, zinc, polyunsaturated fatty acids, arginin and isomers thereof.

19. The composition claimed in claim 4 or claim 18 wherein said folate is selected from the group consisting of folic acid, folinic acid, 5-methyltetrahydrofolic acid, tetrahydrofolic acid and 5-formyltetrahydrofolic acid.

20. A single unit dosage for use in treating an individual with Type II Diabetes consisting of metformin, an ACE inhibitor, and a HGM CoA reductase inhibitor and at least one or more of aspirin, a folate, one or more vitamins and minerals.

## Patentansprüche

1. Zusammensetzung, die als Einzeldosiseinheit formuliert ist, zur Verwendung bei der Behandlung von Diabetes Typ II, Syndrom X und Fettleibigkeit, die ein hypoglykämisches Mittel umfasst, das aus einem Biguanid, einem HGM-CoA-Reduktase-Inhibitor und einem ACE-Inhibitor besteht, wobei die genannte Zusammensetzung in einer Einzeldosiseinheit vorliegt.

2. Zusammensetzung nach Anspruch 1, wobei der genannte ACE-Inhibitor ausgewählt ist aus der Gruppe bestehend aus sulfhydrilhaltigen ACE-Inhibitoren, dicarboxylhaltigen ACE-Inhibitoren und phosphorhaltigen ACE-Inhibitoren.

3. Zusammensetzung nach Anspruch 1, wobei das genannte Statin ausgewählt ist aus der Gruppe bestehend aus Simvastatin, Pravastatin, Lovastatin und Atorvastatin.

4. Zusammensetzung nach Anspruch 1, die ferner wenigstens eines der Folgenden enthält: Folat, Vitamin B6, Vitamin B12, Carotinoid, Vitamin A, Vitamin C, Vitamin D, Vitamin E, Vitamin K, Zink, mehrfach ungesättigte Fettsäuren, Arginin und Isomere davon.

5. Zusammensetzung nach Anspruch 4, wobei die genannte Zusammensetzung Vitamin B6 und Vitamin B12 beinhaltet.

6. Zusammensetzung zur Verwendung bei der Behandlung von Diabetes Typ II, die aus einem hypoglykämischen Biguanid-Mittel, einem lipidsenkenden Mittel und einem ACE-Inhibitor und aus wenigstens einem der Folgenden besteht: einem Anti-Thrombozyten-Wirkstoff, Folat, Vitamin B6, Vitamin B12, Carotinoid, Vitamin A, Vitamin C, Vitamin D, Vitamin E, Vitamin K, Zink, mehrfach ungesättigter Fettsäure, Arginin und einem Arginin-Isomer, die alle in einer Einzeldosiseinheit vorliegen.

7. Zusammensetzung nach Anspruch 6, wobei das genannte hypoglykämische Mittel ausgewählt ist aus der Gruppe bestehend aus Metformin, Pioglitazon, Metformin plus Glyburid, Metformin plus Acarbose, Metformin plus Pioglitazon, Metformin plus Pioglitazon plus Gliburid und Repaglinid plus Pioglitazon.

8. Zusammensetzung in einer Einzeldosiseinheit zur Verwendung bei der Behandlung einer Person mit diagnostiziertem Diabetes Typ II und/oder Syndrom X, polyzystischem Ovarsyndrom und Fettleibigkeit, die ein hypoglykämisches Mittel umfasst, das aus einem Biguanid, einem lipidsenkenden Mittel und einem ACE-Inhibitor besteht.

9. Zusammensetzung nach einem der Ansprüche 2 oder 8, wobei der genannte ACE-Inhibitor ausgewählt ist aus der Gruppe bestehend aus Lisinopril und Ramipril.

10. Zusammensetzung nach Anspruch 8, wobei das genannte blutlipidsenkende Mittel ausgewählt ist aus der Gruppe bestehend aus HMG-CoA-Reduktase-Inhibitoren, Gallensäuremaskierungsmitteln, Probucol und Fibrinsäuremitteln.

11. Zusammensetzung nach Anspruch 10, wobei der genannte HMG-CoA-Reduktase-Inhibitor ausgewählt ist aus der Gruppe bestehend aus Simvastatin, Pravastatin, Lovastatin und Atorvastatin.

12. Zusammensetzung nach einem der Ansprüche 1 oder 8, wobei das genannte hypoglykämische Biguanid-Mittel Metformin ist.

13. Zusammensetzung nach Anspruch 12, die ferner einen Anti-Thrombozyten-Wirkstoff umfasst.

14. Zusammensetzung nach Anspruch 13, wobei der genannte Anti-Thrombozyten-Wirkstoff ausgewählt ist aus der Gruppe bestehend aus Salicylaten, Anti-Thrombozyten-Aggregationsmitteln und Cyclooxygenase-Inhibitoren.

15. Zusammensetzung nach Anspruch 14, wobei das genannte Salicylat ausgewählt ist aus der Gruppe bestehend aus Acetylsalicylsäure und Magnesiumsalicylat.

16. Zusammensetzung nach Anspruch 14, wobei das Anti-Thrombozyten-Aggregationsmittel ausgewählt ist aus der Gruppe bestehend aus Anagrelid, Dipyridamol, Clopidogrel und Ticlopidin.

17. Zusammensetzung nach Anspruch 14, wobei der genannte Cyclooxygenase-Inhibitor ausgewählt ist aus der Gruppe bestehend aus Ibuprofen, Sulindac, Sulindacsulfid, Sulindacsulfon, Flurbiprofen, Indomethacin, Naproxen, Meclofenaminsäure und Piroxicam.

18. Zusammensetzung nach Anspruch 8, wobei das genannte Medikament ferner wenigstens eines der Folgenden umfasst: Folat, Vitamin B6, Vitamin B12, Carotinoid, Vitamin A, Vitamin C, Vitamin D, Vitamin E, Vitamin K, Zink, mehrfach ungesättigte Fettsäuren, Arginin und Isomere davon.

19. Zusammensetzung nach Anspruch 4 oder Anspruch 18, wobei das genannte Folat ausgewählt ist aus der Gruppe bestehend aus Folsäure, Folinsäure, 5-Methyltetrahydrofolsäure, Tetrahydrofolsäure und 5-Formyltetrahydrofolsäure.

20. Einzeldosiseinheit zur Verwendung bei der Behandlung einer Person mit Diabetes Typ II, bestehend aus Metformin, einem ACE-Inhibitor und einem HGM-CoA-Reduktase-Inhibitor sowie Aspirin und/oder Folat, einem oder mehreren Vitamin(en) und Mineral(ien).

## Revendications

1. Composition, formulée en tant que dosage unitaire unique, destinée à être utilisée dans le traitement du Diabète de type II, le Syndrome X et l'obésité, comprenant un agent hypoglycémique consistant en un biguanine, un inhibiteur de l'HGM-CoA réductase, et un inhibiteur ACE, ladite composition dans un dosage unitaire unique.

2. Composition selon la revendication 1, dans laquelle ledit inhibiteur ACE est sélectionné dans le groupe consistant en inhibiteurs ACE contenant un groupe sulfhydryl, en inhibiteurs ACE contenant un groupe dicarboxyle et en inhibiteurs ACE contenant du phosphore.

3. Composition selon la revendication 1, dans laquelle ladite statine est sélectionnée dans le groupe consistant en simvastatine, pravastatine, lovastatine et atorvastatine.

4. Composition selon la revendication 1, comprenant en outre au moins un des composants suivants : un folate, de la vitamine B6, de la vitamine B12, un carotinoïde, de la vitamine A, de la vitamine C, de la vitamine D, de la vitamine E, de la vitamine K, du zinc, des acides gras polyinsaturés, de l'arginine et des isomères de celle-ci.

5. Composition selon la revendication 4, dans laquelle ladite composition comporte de la vitamine B6 et de la vitamine B12.

6. Composition destinée à être utilisée dans le traitement du Diabète de type II consistant en un agent hypoglycémique de biguanide, un hypolipidémiant, et un inhibiteur ACE, et au moins l'un des composants suivants : un antiplaquettaire, un folate, de la vitamine B6, de la vitamine B12, un carotinoïde, de la vitamine A, de la vitamine C, de la vitamine D, de la vitamine E, de la vitamine K, du zinc, des acides gras polyinsaturés, de l'arginine et un isomère d'arginine qui sont tous présents en une dose unitaire unique.

7. Composition selon la revendication 6, dans laquelle ledit agent hypoglycémique est sélectionné dans le groupe consistant en metformine, pioglitazone, métformine plus glyburide, métformine plus acarbose, métformine plus pioglitazone, metformine plus pioglitazone plus glyburide, et répaglinide plus pioglitazone.

8. Composition en un dosage unitaire unique destinée à être utilisée dans le traitement d'un individu diagnostiqué comme souffrant d'au moins l'un du Diabète de type II, du Syndrome X, du syndrome des ovaires polykistiques, et d'obésité comprenant un agent hypoglycémique consistant en un biguanide, un hypolipidémiant et un inhibiteur ACE.

9. Composition selon la revendication 2 ou la revendication 8, dans laquelle ledit inhibiteur ACE est sélectionné dans le groupe consistant en lisinopril et ramipril.

10. Composition selon la revendication 8, dans laquelle ledit hypolipidémiant sanguin est sélectionné dans le groupe consistant en inhibiteurs de l'HMG-CoA réductase, séquestrants d'acide bilaire, probucol et agents d'acide fibrique.

11. Composition selon la revendication 10, dans laquelle ledit inhibiteur de l'HMG-CoA réductase est sélectionné dans le groupe consistant en simvastatine, pravastatine, lovastatine et atorvastatine.

12. Composition selon la revendication 1 ou la revendication 8, dans laquelle ledit agent hypoglycémique de biguanide est la metformine.

13. Composition selon la revendication 12, comprenant en outre un antiplaquettaire.

14. Composition selon la revendication 13, dans laquelle ledit antiplaquettaire est sélectionné dans le groupe consistant en salicylates, agents anti-agrégants plaquettaires, et inhibiteurs de cyclooxygénase.

15. Composition selon la revendication 14, dans laquelle ledit salicylate est sélectionné dans le groupe consistant en acide acétylsalicylique et salicylate de magnésium.

16. Composition selon la revendication 14, dans laquelle ledit agent anti-agrégant plaquettaire est sélectionné dans le groupe consistant en anagrelide, dipyridamole, clopidogrel et ticlopidine.

17. Composition selon la revendication 14, dans laquelle ledit inhibiteur de cyclooxygénase est sélectionné dans le groupe consistant en ibuprofène, sulindac, sulfure de sulindac, sulfone de sulindac, flurbiprofène, indométhacine, naproxène, acide méclofénamique et piroxicam.

18. Composition selon la revendication 8, dans laquelle ledit médicament comprend en outre au moins l'un des composants suivants : un folate, de la vitamine B6, de la vitamine B12, un carotinoïde, de la vitamine A, de la vitamine C, de la vitamine D, de la vitamine E, de la vitamine K, du zinc, des acides gras polyinsaturés, de l'arginine et des isomères de celle-ci.

19. Composition selon la revendication 4 ou la revendication 18, dans laquelle ledit folate est sélectionné dans le groupe consistant en acide folique, acide folinique, acide 5-méthyltétrahydrofolique, acide tétrahydrofolique et acide 5-formyltétrahydrofolique.

20. Dosage unitaire unique destiné à être utilisé pour le traitement d'un individu souffrant de Diabète de type II consistant en metformine, un inhibiteur ACE, et un inhibiteur de l'HGM-CoA réductase et au moins l'un ou plusieurs des composants suivants ; aspirine, folate, un ou plusieurs vitamines et minéraux.
